# EUROPÄISCHE PATENTANMELDUNG

(11) **EP 2 361 599 A1**
(43) Veröffentlichungstag der Anmeldung: **31.08.2011**
(21) Anmeldenummer: 10154304.9
(22) Anmeldetag: 22.02.2010
(51) Int. Cl.: A61J 1/20

(54) **Vorrichtung zum Zuführen oder Entnehmen einer Flüssigkeit in ein oder aus einem Behältnis**

(71) Anmelder: Fresenius Kabi Deutschland GmbH, 61352 Bad Homburg (DE)
(72) Erfinder: Pütter, Harry, 36364, Bad Salzschlirf (DE); Lehmann, Björn, 36251, Bad Hersfeld (DE)
(74) Vertreter: Richly, Erik

(57) **Zusammenfassung**

Die Erfindung betrifft eine Vorrichtung zum Zuführen oder Entnehmen einer Flüssigkeit in ein oder aus einem Behältnis mit einem Einstechdorn (1) und einem mit dem Einstechdorn (1) verbundenen Gehäuse (10, 20, 30), wobei der Einstechdorn (1) einen Flüssigkeitskanal (6) und einen Belüftungskanal (7) aufweist und das Gehäuse (10, 20, 30) eine mit dem Flüssigkeitskanal (6) verbundene Flüssigkeitsfilterkammer (24) und eine mit dem Belüftungskanal (7) verbundene Belüftungsfilterkammer (11) vorgesehen ist. Um eine größere Filterfläche bei minimaler Baugröße zu erreichen, sind erfindungsgemäß die Flüssigkeitsfilterkammer (24) und die Belüftungsfilterkammer (11) in Richtung einer sich entlang des Einstechdorns (1) erstreckenden Längsachse (4) übereinander angeordnet.

## Beschreibung

Die vorliegende Anmeldung betrifft eine Vorrichtung zum Zuführen oder Entnehmen einer Flüssigkeit in ein oder aus einem Behältnis mit einem Einstechdorn und einem mit dem Einstechdorn verbundenen Gehäuse, wobei der Einstechdorn einen Flüssigkeitskanal und einen Belüftungskanal aufweist und in dem Gehäuse eine mit dem Flüssigkeitskanal verbundene Flüssigkeitsfilterkammer zur Aufnahme eines Flüssigkeitsfilters und eine mit dem Belüftungskanal verbundene Belüftungsfilterkammer zur Aufnahme eines Belüftungsfilters vorgesehen ist.

Eine solche Vorrichtung, die auch als Entnahmespike bezeichnet wird, ist aus dem Stand der Technik bekannt. Sie dient in der Medizin- und Labortechnik zum Entnehmen einer Flüssigkeit aus einem Behältnis oder dazu, Flüssigkeiten in Behältnisse zu injizieren. Derartige Flüssigkeiten können Therapeutika wie Heparin oder Zytostatika oder andere pharmazeutisch wirksame Substanzen umfassen. Dabei wird der Einstechdorn in einen Verschluss des entsprechenden Behältnisses eingestochen. Der Belüftungskanal stellt sicher, dass bei der abgedichteten Anordnung des Einstechdorns in dem Verschluss des Behältnisses ein Luftaustausch zwischen dem Inneren und dem Äußeren des Behältnisses abhängig vom sich ändernden Füllstand der Flüssigkeit erfolgt.

Weiterhin ist es erforderlich, dass die von außen in das Behältnis gelangende, oder beim Zuführen nach außen abgeführte Luft und ggf. die zugeführte oder entnommene Flüssigkeit gefiltert wird. Filter bewirken hierbei, unerwünschte Partikel und weitere Feststoffe einschließlich biologischer Elemente von der Luft oder der Flüssigkeit zu trennen.

Aus der Druckschrift DE 38 20 204 C2 ist eine oben beschriebene Vorrichtung bekannt, bei der eine erste Kammer für einen Filter, welche mit dem Flüssigkeitskanal verbunden ist, und eine zweite Kammer für einen weiteren Filter, welche mit dem Belüftungskanal verbunden ist, in einer Trägerplatte gebildet sind. Die erste und die zweite Kammer sind in der Trägerplatte nebeneinander angeordnet, wobei eine Kammer einen C-förmigen Querschnitt und die andere Kammer einen rechteckigen Querschnitt aufweist und die eine Kammer die andere umschließt.

Auch bei dem aus der Druckschrift EP 1 192 927 B1 bekannten Entnahmespike sind eine erste Filterkammer und eine zweite Filterkammer in einem plattenförmigen Gehäuse ausgebildet. Die erste Filterkammer ist mit einem Flüssigkeitsfilter und die zweite Filterkammer mit einem Luftfilter versehen. Auch diese Filterkammern sind in dem Gehäuse nebeneinander angeordnet, wobei der Flüssigkeitskanal mit der ersten Filterkammer und der Luftkanal mit der zweiten Filterkammer in Verbindung stehen.

Der Nachteil der bekannten Lösungen besteht darin, dass mit einer sehr kleinen Filterfläche gearbeitet werden muss, da die Summe der Querschnittsbreite der nebeneinander liegenden Filterkammern die Breite des Entnahmespike-Gehäuses bestimmt. Alternativ müssen die Abmessungen des Entnahmespikes vergrößert werden, was häufig nicht gewünscht ist und auch die Materialkosten erhöht.

Die Aufgabe der vorliegenden Erfindung besteht somit darin, eine Vorrichtung zum Zuführen oder Entnehmen einer Flüssigkeit zu schaffen, welche eine größere Filterfläche zur Verfügung stellt.

Die obige Aufgabe wird gelöst durch eine Vorrichtung, bei der die Flüssigkeitsfilterkammer und die Belüftungsfilterkammer in Richtung einer sich entlang des Einstechdorns erstreckenden Längsachse übereinander (oder, anders ausgedrückt, hintereinander) angeordnet sind. Diese Anordnung von Flüssigkeitsfilterkammer und Belüftungsfilterkammer bedeutet auch, dass die beiden Kammern entlang des Fließweges der Flüssigkeit hintereinander oder nacheinander angeordnet sind.

Die obige erfindungsgemäße Vorrichtung hat den Vorteil, dass jedem Filter fast die gesamte Breite der Vorrichtung bzw. des Gehäuses als Filterfläche zur Verfügung steht, da aufgrund der erfindungsgemäßen Anordnung von Flüssigkeitsfilterkammer und Belüftungsfilterkammer jede Kammer im Wesentlichen die gesamte Breite der Vorrichtung bzw. des Gehäuses nutzen kann. Hieraus ergibt sich in besonders vorteilhafter Weise, dass Filter mit einer kleineren Maschenweite eingesetzt werden können, da aufgrund der Filtergröße auch bei kleinerer Maschenweite ein ausreichender Durchsatz an Luft oder Flüssigkeit erzielt wird. Hierdurch kann eine höhere Druckstabilität gegen Flüssigkeitsdurchbruch erreicht werden, so dass eine Kontamination durch austretende Flüssigkeit vermieden wird, ohne größere Einbußen in der Leistung in Kauf nehmen zu müssen.

Vorzugsweise sind hierbei die Flüssigkeitsfilterkammer und die Belüftungsfilterkammer im Wesentlichen parallel zueinander angeordnet. Dies bedeutet, dass eine quer zur Längsachse des Einstechdorns durch die Flüssigkeitsfilterkammer verlaufende Mittellinie und eine quer zur Längsachse des Einstechdorns durch die Belüftungsfilterkammer verlaufende Mittellinie sich in einem Winkel von höchstens 30°, vorzugsweise von höchstens 10°, besonders bevorzugt etwa parallel zueinander in einem Winkel von höchstens 5°, zueinander erstrecken. Die Mittellinie der Flüssigkeitsfilterkammer und die Mittellinie der Belüftungsfilterkammer bilden jeweils Achsen, die entlang der größten Ausdehnung der jeweiligen Kammer verlaufen. Die Mittellinie jeder Kammer kann dabei auch eine Mittellinie der in der jeweiligen Kammer angeordneten Filtermembran bilden.

In einer besonders bevorzugten Ausgestaltung der vorliegenden Erfindung ist der Flüssigkeitskanal abgedichtet gegenüber der Belüftungsfilterkammer durch diese und/oder der Belüftungskanal abgedichtet gegenüber der Flüssigkeitsfilterkammer durch diese hindurch geführt. Alternativ hierzu ist es auch möglich, dass einer der Kanäle seitlich an einer der Filterkammern vorbei geführt wird.

Bei der vorliegenden Erfindung beinhaltet der Begriff "Hindurchführen" auch, dass ein Kanal an der jeweiligen Kammer vorbei geführt wird, derart dass sich die jeweilige Kammer, durch die der Kanal "hindurch geführt" wird, lediglich auf einer Seite des Kanals erstreckt. Dies bedeutet, dass der Begriff "Hindurchführen" bei der vorliegenden Erfindung derart weit verstanden wird, dass der Kanal, der durch die Kammer hindurchgeführt wird, entlang der gesamten Ausdehnung der Kammer in Richtung der Längsachse des Einstechdorns verläuft. Die Ausdehnung der Kammer in Richtung der Längsachse des Einstechdorns kann auch als Höhe der Kammer bezeichnet werden.

Diese Ausführungsform gewährleistet insbesondere dann, wenn die Durchführung des Flüssigkeitskanals durch die Belüftungsfilterkammer und/oder die Durchführung des Belüftungskanals durch die Flüssigkeitsfilterkammer etwa im mittigen Bereich des Gehäuses angeordnet ist, einen einfachen Aufbau, der auch produktionstechnisch vorteilhaft ist.

In einer vorteilhaften Weiterbildung der vorliegenden Erfindung ist die Belüftungsfilterkammer zur Aufnahme eines Membranfilters ausgebildet, welcher zumindest die Form eines Abschnitts (oder Segments) eines Kreisrings aufweist. Dieser Aufbau der Belüftungsfilterkammer ist sehr einfach und lässt sich produktionstechnisch einfach umsetzen. Zudem ergeben sich auch bei der Montage einer solchen Vorrichtung Vorteile, da die Anordnung eines kreisringförmigen Membranfilters in einer entsprechenden Ausnehmung oder Vertiefung des Gehäuses besonders genau erfolgen kann. Erfindungsgemäß muss ein Membranfilter lediglich im Wesentlichen die Form zumindest eines Abschnitts eines Kreisrings aufweisen, d.h. auch ovale und/oder achsversetzte Ausführungsformen sind möglich. Zudem kann auch die jeweilige Filterkammer z. B. eine innere Öffnung aufweisen, die oval und/oder achsversetzt ist.

Es ist ferner bevorzugt, wenn in der Belüftungsfilterkammer ein Belüftungsfilter, vorzugsweise ein im Wesentlichen kreisringförmiger Membranfilter, und/oder in der Flüssigkeitsfilterkammer ein Flüssigkeitsfilter, vorzugsweise ein im Wesentlichen kreisförmiger Membranfilter angeordnet ist, welche vorzugsweise konzentrisch angeordnet sind. Wenn in beiden Kammern Filter vorgesehen sind, können die Flüssigkeit und die Luft gleichzeitig gefiltert werden. Die konzentrische Anordnung der Bestandteile der erfindungsgemäßen Vorrichtung bewirkt eine Verbesserung der automatischen Montage, da die Montagerichtung konstant bleibt. Die Maschenweite der Belüftungsfiltermembran kann 0,02 µm bis 3 µm betragen, bevorzugt 0,2 µm bis 3 µm. Die Maschenweite der Flüssigkeitsfiltermembran kann 1 µm bis 15 µm betragen, bevorzugt 5 µm.

In einem weiteren bevorzugten Ausführungsbeispiel weist die Ober- und/oder Unterseite der Flüssigkeitsfilterkammer und/oder die Ober- und/oder Unterseite der Belüftungsfilterkammer Stützrippen auf. In einer bevorzugten Ausführungsform sind diese so angeordnet sind, das die ggf. in der Flüssigkeitsfilterkammer oder der Belüftungsfilterkammer gegenüber liegenden Stützrippen schräg und/oder versetzt zueinander verlaufen. Hierdurch wird gewährleistet, dass sich nicht jeweils Spitzen oder Berge bzw. Täler der Stützrippen gegenüber stehen. So wird die gesamte Filtergröße optimal zur Filterung der Luft bzw. Flüssigkeit ausgenutzt und das Totvolumen der Vorrichtung ist optimiert, so dass ein nahezu kompletter Luft- oder Flüssigkeitsaustausch erfolgt.

Vorteilhaft weist die Flüssigkeitskammer und/oder Belüftungsfilterkammer ferner in einer Weiterbildung der Erfindung jeweils an ihrer Ober- und/oder Unterseite strahlenförmig verlaufende Vertiefungen auf. Diese als Kanäle ausgebildeten Vertiefungen verlaufen ausgehend von einem mittleren Bereich, in dem die Flüssigkeit oder die Luft die jeweilige Kammer durch eine Öffnung wieder verlässt, strahlenförmig und bewirken eine gleichmäßige Verteilung der Luft bzw. der Flüssigkeit über die gesamte Oberfläche des jeweiligen Filters.

Es ist weiterhin von Vorteil, wenn das Gehäuse aus mindestens zwei vorzugsweise übereinander angeordneten Teilen zusammengesetzt ist, wobei besonders bevorzugt ein Gehäuseunterteil, ein Gehäusemittelteil und ein Gehäuseoberteil vorgesehen sind. Bevorzugt sind die Belüftungsfilterkammer durch das Gehäuseunterteil und das Gehäusemittelteil und die Flüssigkeitsfilterkammer durch das Gehäusemittelteil und das Gehäuseoberteil gebildet.

Der modulare Aufbau des Gehäuses aus mehreren Teilen ermöglicht, dass sehr leicht verschiedene Varianten der erfindungsgemäßen Vorrichtung, beispielsweise mit unterschiedlicher Konstruktion des Einstechdorns oder einem veränderten Anschlussgehäuse mit oder ohne Ventil realisiert werden können, ohne dass zugleich die gesamte Vorrichtung geändert werden muss. Hierdurch werden Herstellungskosten gespart. Durch den modularen Aufbau ist auch gewährleistet, dass je nach Bedarf verschiedene Filter eingesetzt werden können oder auch auf einen Filter, beispielsweise in der Flüssigkeitsfilterkammer, verzichtet werden kann. Diese Entscheidungen können im Produktionsprozess während der Herstellung der erfindungsgemäßen Vorrichtung kurzfristig getroffen werden. Es kann somit schnell und flexibel auf entsprechende Spezifikationen des Kunden reagiert werden.

Bevorzugt ist das erste Teil, vorzugsweise das Gehäuseunterteil, mit dem Einstechdorn verbunden und ein weiteres Teil, vorzugsweise das Gehäuseoberteil, mit einem Anschlussgehäuse verbunden. Besonders bevorzugt ist dabei das erste Teil, vorzugsweise das Gehäuseunterteil, mit dem Einstechdorn einstückig ausgebildet.

Eine vorteilhafte Steuerung der zugeführten oder abgeführten Flüssigkeit wird erreicht, indem das Anschlussgehäuse ein Ventil, vorzugsweise mit einer selbstverschließenden Ventilplatte und einem Ventilbetätiger, aufweist.

Produktionsbedingte Vorteile ergeben sich auch, wenn das Gehäuse zumindest abschnittsweise radialsymmetrisch ausgebildet ist.

Um mit der erfindungsgemäßen Vorrichtung auch Zytostatika einem Behältnis zuführen oder entnehmen zu können, ist es von Vorteil, wenn die Vorrichtung zumindest in den die Flüssigkeit führenden Bereichen aus ABS (Acrylnitril-Butadien-Styrol-Copolymerisat) besteht. Als weitere Materialien können für die erfindungsgemäße Vorrichtung beispielsweise Polystyrol, Hart-PVC (Hart-Polyvinylchlorid), PC (Polycarbonat), SAN (Styro-Acrylnitril), Polypropylen und/oder MABS (Methyl-Methacrylat-Acrylnitril-Butadien-Styrol) verwendet werden.

Weiterbildungen, Vorteile und Anwendungsmöglichkeiten der Erfindung ergeben sich auch aus der nachfolgenden Beschreibung eines Ausführungsbeispiels und den Figuren. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den Ansprüchen oder deren Rückbeziehung.

Es zeigen schematisch:
- Fig. 1: eine erfindungsgemäße Vorrichtung in einer perspektivischen Ansicht von oben,
- Fig. 2: die erfindungsgemäße Vorrichtung gemäß Figur 1 in einer perspektivischen Ansicht von unten,
- Fig. 3: die erfindungsgemäße Vorrichtung gemäß Figur 1 in einer ersten Ansicht von der Seite,
- Fig. 4: die Vorrichtung gemäß Figur 1 in einer zweiten Ansicht von der Seite,
- Fig. 5: die Vorrichtung gemäß Figur 1 in einer Querschnittdarstellung entlang der Linie A-A (siehe Figur 4) mit Filtern,
- Fig. 6: die Darstellung gemäß Figur 5 ohne Filter,
- Fig. 7: eine Explosionsdarstellung der erfindungsgemäßen Vorrichtung gemäß Figur 1 in einer perspektivischen Ansicht von oben und
- Fig. 8: eine Explosionsdarstellung der erfindungsgemäßen Vorrichtung gemäß Figur 1 in einer perspektivischen Ansicht von unten.

Die erfindungsgemäße Vorrichtung zum Zuführen oder Entnehmen einer Flüssigkeit in ein oder aus einem Behältnis wird im Folgenden in ihrer Funktion als Vorrichtung zum Entnehmen einer Flüssigkeit erläutert und in der unten stehenden Beschreibung kurz als Entnahmespike bezeichnet. Mit gleichem Aufbau kann der Entnahmespike auch als Vorrichtung zum Zuführen von Flüssigkeit in ein Behältnis verwendet werden.

Der Entnahmespike weist an seinem unteren Ende einen Einstechdorn 1 auf, mit welchem dieser in den Verschluss eines nicht dargestellten Behältnisses eingestochen werden kann.

Das Gehäuse setzt sich aus einem Gehäuseunterteil 10, einem Gehäusemittelteil 20 und einem Gehäuseoberteil 30 zusammen, welche entlang einer sich entlang des Einstechdorns 1 erstreckenden Längsachse 4 in dieser Reihenfolge übereinander angeordnet sind. Der Einstechdorn 1 ist mit dem Gehäuseunterteil 10 einstückig ausgebildet. An dem Gehäuseoberteil 30 ist ein Anschlussgehäuse 40 befestigt, dessen oberes Ende durch eine Kappe 50 verschlossen werden kann. Grundsätzlich können das Gehäuseoberteil 30 und das Anschlussgehäuse 40 auch einstückig ausgebildet sein, insbesondere bei Ausführungsformen ohne ein unten näher beschriebenes Ventil.

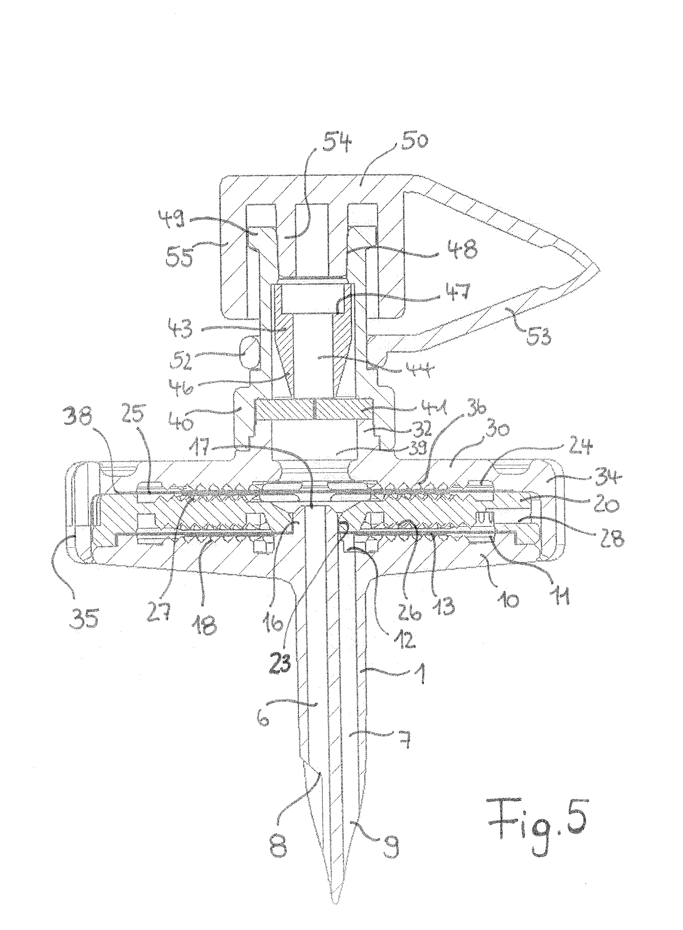

Das Gehäuseunterteil 10 und das Gehäusemittelteil 20, das Gehäusemittelteil 20 und das Gehäuseoberteil 30 sowie das Gehäuseoberteil 30 und das Anschlussgehäuse 40 sind vorzugsweise miteinander verschweißt, verklebt oder auf sonstige Weise abgedichtet aneinander befestigt. Dabei können zwischen dem Gehäuseunterteil 10 und dem Gehäusemittelteil 20 in Fig. 5 angedeutete Führungsbereiche vorgesehen sein.

Parallel zu der Längsachse 4 des Einstechdorns 1 verlaufen in diesem ein Flüssigkeitskanal 6 und ein Belüftungskanal 7. Der Flüssigkeitskanal 6 endet an dem dem Gehäuse gegenüber liegenden Ende des Einstechdorns 1 in einer Flüssigkeitseintrittsöffnung 8 und der Belüftungskanal 7 endet an dem gleichen Ende des Einstechdorns 1 in einer Luftaustrittsöffnung 9.

Zwischen dem Gehäuseunterteil 10 und dem Gehäusemittelteil 20 ist eine Belüftungsfilterkammer 11 ausgebildet, welche zur Anordnung einer kreisringförmigen Belüftungsfiltermembran 13 mit einer Maschenweite von 0,02 µm bis 3 µm dient. Mit ihrer durchgehenden kreisförmigen Öffnung 15 ist die Belüftungsfiltermembran 13 um einen zylindrischen vorspringenden Abschnitt 16 des Gehäuseunterteils 10 gelegt. Hierdurch kann eine genaue Platzierung der Belüftungsfiltermembran bewirkt werden. Der Belüftungskanal 7 endet an der Oberseite des Gehäuseunterteils 10 mit einer Öffnung 12, so dass der Belüftungskanal 7 mit der Belüftungsfilterkammer 13 verbunden ist.

Der Flüssigkeitskanal 6 erstreckt sich in dem Gehäuseunterteil 10 bis in den zylindrischen vorspringenden Abschnitt 16, welcher von dem Gehäuseunterteil 10 nach oben vorsteht. Der zylindrische Abschnitt 16 ist abgedichtet oder verschweißt in einer entsprechenden durchgehenden Öffnung 23 des Gehäusemittelteils 20 angeordnet. Mittels des zylindrischen Abschnitts 16 wird der Flüssigkeitskanal 6 durch die Belüftungsfilterkammer 11 hindurchgeführt, so dass dieser gegenüber der Belüftungsfilterkammer 11 abgedichtet ist.

Der Flüssigkeitskanal 6 endet an dem dem Einstechdorn 1 gegenüber liegenden oberen Ende bzw. am Ende des zylindrischen Abschnitts 16 mit einer Flüssigkeitsaustrittsöffnung 17, welche mit einer Flüssigkeitsfilterkammer 24 verbunden ist. Die Flüssigkeitsfilterkammer 24 ist zwischen dem Gehäusemittelteil 20 und dem Gehäuseoberteil 30 ausgebildet. In der Flüssigkeitsfilterkammer 24 ist eine kreisförmige Flüssigkeitsfiltermembran 25 mit einer Maschenweite von 1 µm bis 15 µm angeordnet. Die Flüssigkeitsfiltermembran kann für bestimmte Anwendungen des Entnahmespikes jedoch auch entfallen, falls z.B. die Gefahr besteht auch die Wirkstoffe eines Medikaments zurückzuhalten. Die Flüssigkeitsfiltermembran hat die Aufgabe, Feststoffe aus der Flüssigkeit herauszufiltern.

Die Flüssigkeit dringt durch die im Einstechdorn ausgebildete Flüssigkeitseintrittsöffnung 8 in den Flüssigkeitskanal 6 des Einstechdorns 1 ein und gelangt dann durch die Öffnung 17 in dem zylindrischen Abschnitt 16 in die Flüssigkeitsfilterkammer 24. Die Flüssigkeitsfilterkammer 24 ist über einen durchgehenden Kanal 39 des Gehäuseoberteils 30 mit einem durchgehenden Kanal 44 des Anschlussgehäuses 40 verbunden, welche ein unten beschriebenes Ventil aufweist. Die Flüssigkeit gelangt somit nach dem Passieren der Flüssigkeitsfiltermembran 25 in den Kanal 44. In dem oberen Ende des durchgehenden Kanals 44 des Anschlussgehäuses 40 kann beispielsweise eine Spritze (nicht dargestellt) angeordnet sein, welche die Flüssigkeit aufnimmt. Die Kanäle 39 und 44 verlaufen entlang der Längsachse 4. Alternativ kann nach einer Variante der Erfindung eine Umlenkung im Bereich der Kammer oberhalb des Flüssigkeitsfilters vorgesehen sein, d.h. es existiert kein durchgehender Kanal, sondern eine Prallplatte.

Das Gehäuseoberteil 30 weist im Bereich seines seitlichen Randes 34 drei durchgehende Belüftungsöffnungen 35 auf, durch welche die Luft von außen in den Entnahmespike eingesaugt wird, sobald in dem Behältnis, in das der Entnahmespike abdichtend eingestochen ist, ein Unterdruck durch die Entnahme von Flüssigkeit entsteht. Die Luft gelangt durch drei weitere seitliche und durchgehende Lufteintrittsöffnungen 28 in dem Gehäusemittelteil 20 und durch die Belüftungsfilterkammer 11 hindurch in den Belüftungskanal 7 und tritt durch die Luftaustrittsöffnung 9 in das Behältnis (nicht dargestellt) wieder aus. Die Erfindung ist jedoch nicht auf diese spezielle Gestaltung des Ortes und der Art der Gehäuseöffnung beschränkt, vielmehr können die Lufteintritts- und -austrittsöffnungen auch an anderen Stellen des Gehäuses vorgesehen sein. In der Belüftungsfilterkammer 11 wird die eindringende Luft durch eine Belüftungsfiltermembran 13 gefiltert, so dass keine Feststoffe wie Partikel oder Bakterien, die größer als die Maschenweite des Belüftungsfilters 13 sind, in das Behältnis gelangen können.

An der Unterseite der Belüftungsfilterkammer 11 weist das Gehäuseunterteil 10 Stützrippen 18 auf, welche im Wesentlichen einen dreieckigen Querschnitt aufweisen, wobei andere Querschnittsformen ebenfalls denkbar sind. Analoge Stützrippen 26, 27 und 36 sind auch an der Oberseite der Belüftungsfilterkammer 11 an dem Gehäusemittelteil 20, an der Unterseite der Flüssigkeitsfilterkammer 24 ebenfalls an dem Gehäusemittelteil 20 sowie an der Oberseite der Flüssigkeitsfilterkammer 24 an dem Gehäuseoberteil 30 ausgebildet. Im Bereich der Stützrippen 18, 26, 27, 36 wird die jeweilige Filtermembran 13, 25 von den Spitzen der Stützrippen 18, 26, 27, 36 gehalten.

In der Belüftungsfilterkammer 11 sind ferner an der Oberseite des Gehäuseunterteils 10 und an der Unterseite des Gehäusemittelteils strahlenförmig verlaufende Vertiefungen 19, 29 in Form von Kanälen vorgesehen, welche von dem Bereich des in etwa mittig angeordneten zylindrischen Abschnitts 16 ausgehen. Analoge Vertiefungen 29, 37 sind auch in der Flüssigkeitsfilterkammer 24 an der Oberseite des Gehäusemittelteils 20 und der Unterseite des Gehäuseoberteils 30 angeordnet. Die Vertiefungen 19, 29, 37 bewirken eine gleichmäßige Verteilung der Luft bzw. der Flüssigkeit über die gesamte Fläche der Belüftungsfiltermembran 13 und der Flüssigkeitsfiltermembran 25. Sie bilden Kanäle zwischen den Stützrippen 18, 26, 27, 36.

Sowohl die Belüftungsfiltermembran 13 als auch die Flüssigkeitsfiltermembran 25 erstrecken sich über fast die gesamte Breite des Gehäuses. Demzufolge steht eine verglichen mit dem oben erläuterten Stand der Technik große Fläche zur Filterung der Luft bzw. der Flüssigkeit bei minimaler Baugröße des Entnahmespikes zur Verfügung. Folglich können Filter mit kleiner Maschenweite, beispielsweise mit einer Maschenweite von 0,2 µm statt 3 µm, verwendet werden, um die Druckstabilität gegen über Flüssigkeitsaustritt bei unsachgemäßer Bedienung vorzubeugen.

Die kleine Baugröße des erfindungsgemäßen Entnahmespikes wird auch deshalb erreicht, da eine Mittellinie 14 der Belüftungsfilterkammer 11 und eine Mittellinie 22 der Flüssigkeitsfilterkammer 24 parallel zueinander angeordnet sind und so insgesamt einen minimalen Rauminhalt einnehmen. Die Mittellinie 14 der Belüftungsfilterkammer 11 und die Mittellinie 22 der Flüssigkeitsfilterkammer 24 verlaufen senkrecht zur Längsachse 4 und bilden gleichzeitig eine Mittellinie der Belüftungsfiltermembran 13 und eine Mittellinie der Flüssigkeitsfiltermembran 25.

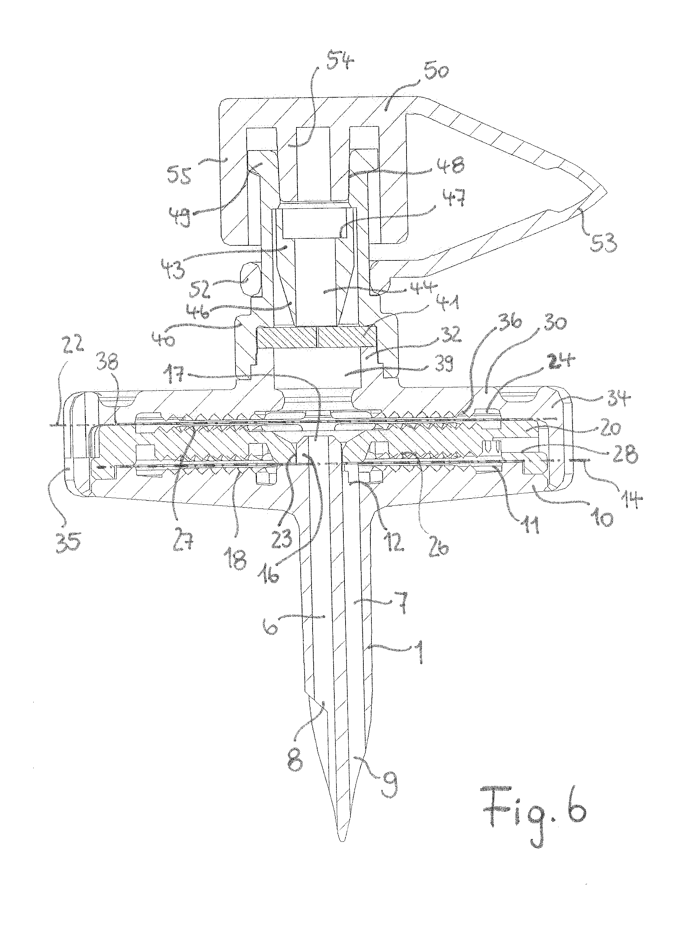

Das Gehäuseoberteil 30 ist in dem oberen, dem Einstechdorn 1 gegenüber liegenden Abschnitt als Anschlussstutzen 32 ausgebildet, mit dem das Anschlussgehäuse 40, beispielsweise mittels einer Schweiß- oder Klebeverbindung, mit dem Gehäuseoberteil 30 verbunden ist oder einstückig ausgeführt ist. Oberhalb des Anschlussstutzens 32 des Gehäuseoberteils 30 ist in Kanal 44 des Anschlussgehäuses 40 eine Ventilplatte 41 angeordnet, welche aus einem Elastomermaterial besteht und zwischen einem Absatz in der Innenoberfläche des Anschlussgehäuses 40 und der Stirnseite des Anschlussstutzens 32 eingespannt ist. Die Ventilplatte 41 weist eine Schlitz- oder Öffnungsstruktur auf. Sie ist selbstschließend, das heißt ohne Einwirkung einer mechanischen Betätigung durch Konnektion am Anschlustutzen begibt sie sich in die in den Figuren 5 und 6 dargestellte Schließposition. Der oberhalb der Ventilplatte 41 angeordnete Ventilbetätiger 43 ist ein rohrförmiges Teil mit einem am unteren Ende vorgesehenen Kegelstumpf 46. An der Innenoberfläche des Ventilbetätigers 43 befinden sich ein nach innen abstehender Vorsprung 47. Beim Einführen beispielsweise einer Spritze in das Anschlussgehäuse 40 stößt das eindringende Teil der Spritze gegen den Vorsprung 47 des Ventilbetätigers 43, welcher daraufhin in dem Anschlussgehäuse 40 nach unten verschoben wird. Durch die dadurch erzeugte Kraftwirkung wird die Ventilplatte 41 aufgedrückt und zwangsweise im Öffnungszustand gehalten, so lange das eindringende Teil der Spritze eine nach unten wirkende Kraft auf den Vorsprung 47 ausübt. Ist dies nicht mehr der Fall, bewegt sich der Ventilbetätiger 43 unter der Federwirkung der elastischen Ventilplatte 41 in die Ausgangslage zurück und das Ventil schließt von selbst.

Die Kappe 50 ist mittels eines um den Hals des Anschlussgehäuses 40 liegenden Rings 52 an dem Anschlussgehäuse 40 befestigt. Der Ring 52 ist mit einem Kniegelenkarm 53 verbunden, welcher den Deckel 50 bei Öffnen bzw. Schließen führt, wodurch sich ein unbeabsichtigtes Spritzen der Flüssigkeit vermeiden lässt. An dem Deckel 50 ist ferner ein zylindrisches Verschlussteil 54 vorgesehen, welches in den Innenkonus 48 des oberen Endes des Anschlussgehäuses 40 eindringt und dieses abdichtend abschließt. Ein ebenfalls an dem Deckel 50 vorgesehenes äußeres zylindrisches Verschlussteil 55 umfasst am oberen Ende des Anschlussgehäuses 40 vorgesehene Vorsprünge 49. Die Vorsprünge 49 verhindern gleichzeitig ein Herabgleiten des Rings 52 vom Hals des Anschlussgehäuses 40.

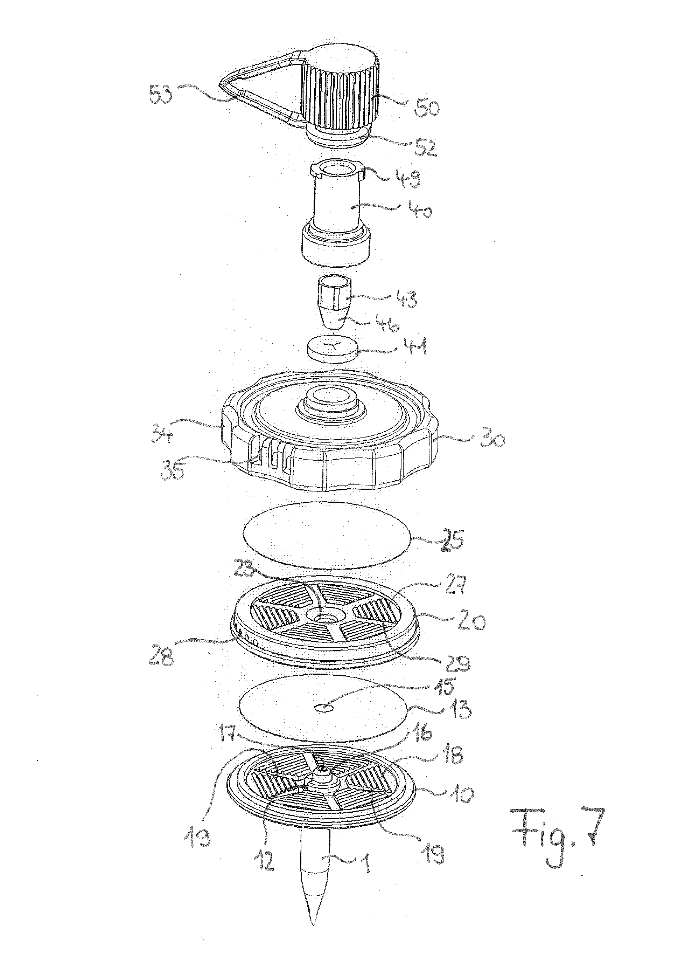

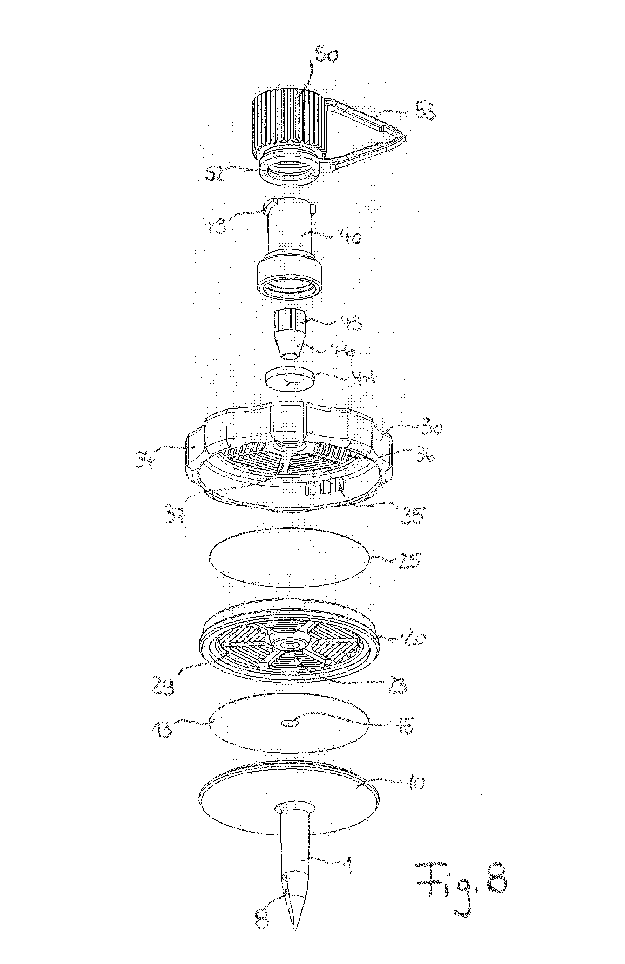

Insbesondere aus den Figuren 7 und 8 ist entnehmbar, dass die Teile des erfindungsgemäßen Entnahmespikes im Wesentlichen konzentrisch zueinander angeordnet sind. Diese Anordnung ermöglicht eine einfache und genaue Montage der Einzelteile im Herstellungsprozess, so dass die Produktion kostengünstig ist.

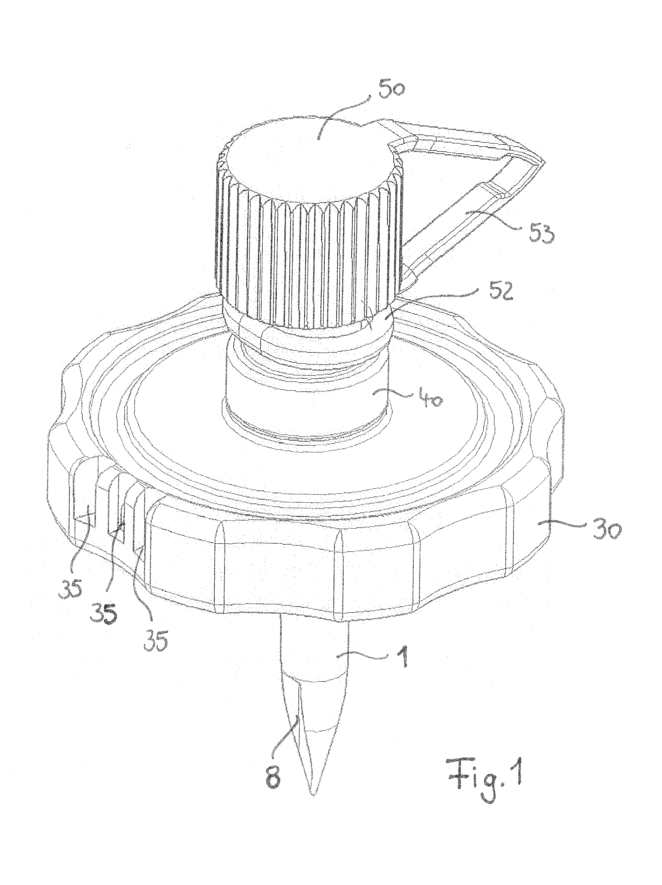

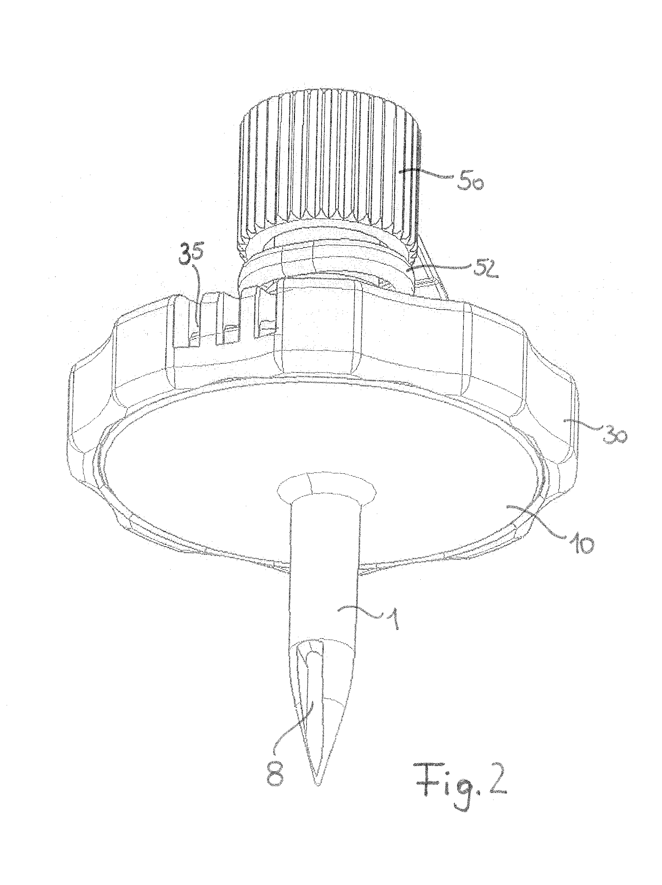

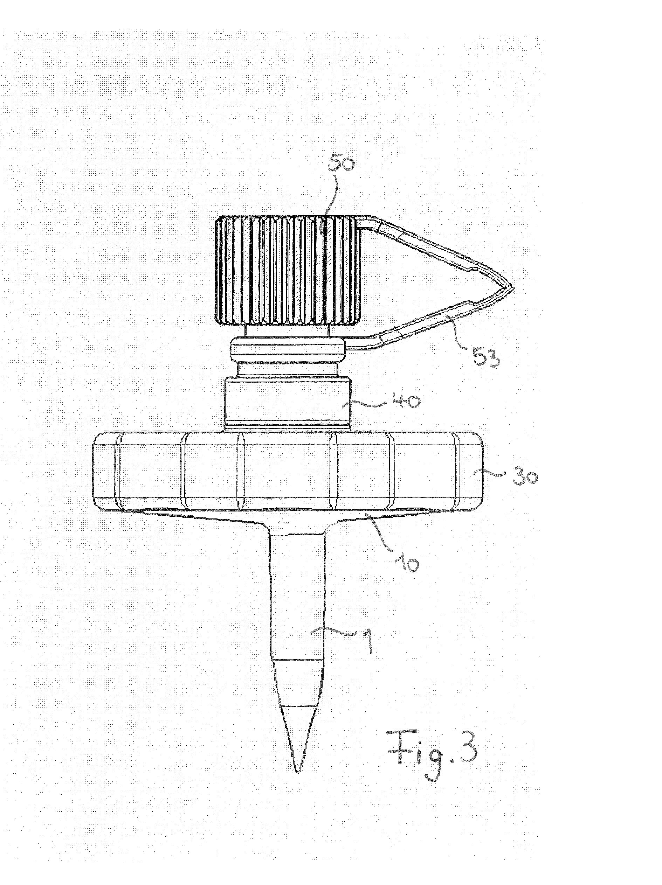

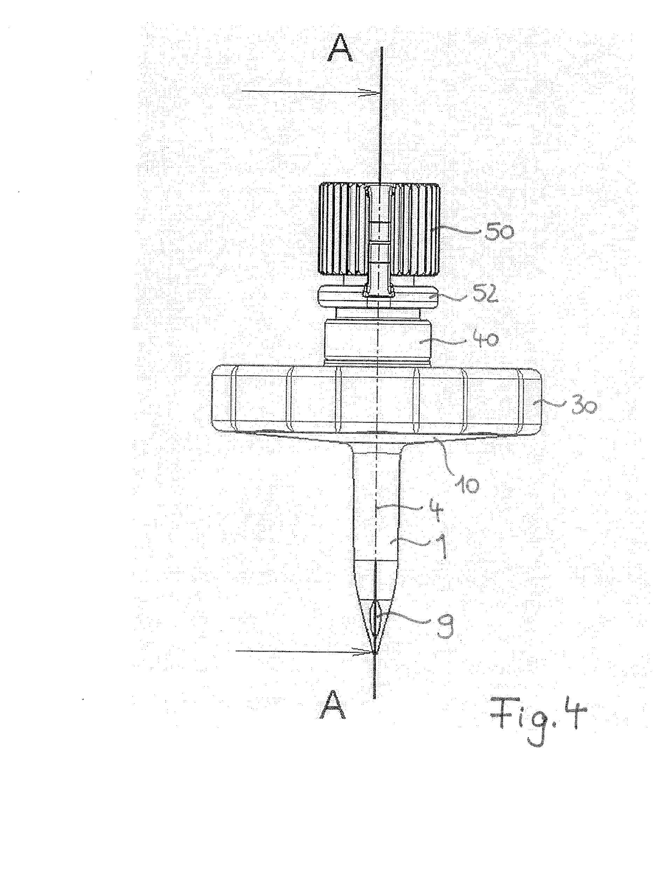

Das oben geschilderte Ausführungsbeispiel umfasst die Variante, in der die Belüftungsfilterkammer 11 unterhalb der Flüssigkeitsfilterkammer 25 angeordnet ist, wenn der Entnahmespike in der in den Figuren 1 bis 8 dargestellten Weise ausgerichtet wird. Es ist jedoch auch möglich, die Flüssigkeitsfilterkammer unterhalb der Belüftungsfilterkammer vorzusehen. In diesem Fall muss sowohl der Flüssigkeitskanal durch die Belüftungsfilterkammer als auch der Belüftungskanal durch die Flüssigkeitsfilterkammer hindurch im Sinne der vorliegenden Erfindung geführt werden. Diese Lösung ist demnach aufwendiger als die anhand der Figuren geschilderte Variante, wird aber dennoch von der vorliegenden Erfindung erfasst.

In der in den Figuren dargestellten Ausführungsform ist die Achse des Einstechdorns 1 nicht identisch mit der Rotationsachse der anderen Bauteile. Vielmehr liegen diese Bauteile alle in Achse mit der Achse des Flüssigkeitskanals 6, während der Dorn 1 hierzu asymmetrisch angeordnet ist. Grundsätzlich kann jedoch auch der Flüssigkeitskanal 6 perspektivisch noch zu den anderen Bauteilen verschoben werden, so dass deren Längsachsen bspw. mit der des Einstechdorns 1 zusammenfallen.

### Bezugszeichenliste:

- 1: Einstechdorn
- 4: Längsachse
- 6: Flüssigkeitskanal
- 7: Belüftungskanal
- 8: Flüssigkeitseintrittsöffnung
- 9: Luftaustrittsöffnung
- 10: Gehäuseunterteil
- 11: Belüftungsfilterkammer
- 12: Öffnung
- 13: Belüftungsfiltermembran
- 14: Mittellinie
- 15: Öffnung
- 16: zylindrischer Abschnitt
- 17: Flüssigkeitsaustrittsöffnung
- 18: Stützrippe
- 19: Vertiefung
- 20: Gehäusemittelteil
- 22: Mittellinie
- 23: Öffnung
- 24: Flüssigkeitsfilterkammer
- 25: Flüssigkeitsfiltermembran
- 26: Stützrippe
- 27: Stützrippe
- 28: Lufteintrittsöffnung
- 29: Vertiefung
- 30: Gehäuseoberteil
- 32: Anschlussstutzen
- 34: Rand
- 35: Belüftungsöffnung
- 36: Stützrippe
- 37: Vertiefung
- 38: kreisförmige Vertiefung
- 39: Kanal
- 40: Anschlussgehäuse
- 41: Ventilplatte
- 43: Ventilbetätiger
- 44: Kanal
- 46: Kegelstumpf
- 47: Vorsprung
- 48: Innenkonus
- 49: Vorsprung
- 50: Kappe
- 52: Ring
- 53: Kniegelenkarm
- 54: inneres zylindrisches Verschlussteil
- 55: äußeres zylindrisches Verschlussteil

## Patentansprüche

1. Vorrichtung zum Zuführen oder Entnehmen einer Flüssigkeit in ein oder aus einem Behältnis mit einem Einstechdorn (1) und einem mit dem Einstechdorn (1) verbundenen Gehäuse (10, 20, 30), wobei der Einstechdorn (1) einen Flüssigkeitskanal (6) und einen Belüftungskanal (7) aufweist und das Gehäuse (10, 20, 30) eine mit dem Flüssigkeitskanal (6) verbundene Flüssigkeitsfilterkammer (24) und eine mit dem Belüftungskanal (7) verbundene Belüftungsfilterkammer (11) vorgesehen ist, **dadurch gekennzeichnet, dass** die Flüssigkeitsfilterkammer (24) und die Belüftungsfilterkammer (11) in Richtung einer sich entlang des Einstechdorns (1) erstreckenden Längsachse (4) übereinander angeordnet sind.

2. Vorrichtung nach Anspruch 1, **dadurch gekennzeichnet, dass** der Flüssigkeitskanal (6) abgedichtet gegenüber der Belüftungsfilterkammer (11) durch diese und/oder der Belüftungskanal (7) abgedichtet gegenüber der Flüssigkeitsfilterkammer (24) durch diese oder seitlich an dieser vorbei geführt ist.

3. Vorrichtung nach einem der vorhergehenden Ansprüche, **dadurch gekennzeichnet, dass** die Belüftungsfilterkammer (11) zur Aufnahme eines Membranfilters (13) ausgebildet ist, welcher im Wesentlichen die Form zumindest eines Abschnitts eines Kreisrings aufweist.

4. Vorrichtung nach einem der vorhergehenden Ansprüche, **dadurch gekennzeichnet, dass** in der Belüftungsfilterkammer (11) ein Belüftungsfilter, vorzugsweise ein im Wesentlichen kreisringförmiger Membranfilter (13), und/oder in der Flüssigkeitsfilterkammer (24) ein Flüssigkeitsfilter, vorzugsweise ein im Wesentlichen kreisförmiger Membranfilter (25), vorgesehen sind, welche besonders bevorzugt konzentrisch zueinander angeordnet sind.

5. Vorrichtung nach einem der vorhergehenden Ansprüche, **dadurch gekennzeichnet, dass** die Ober- und/oder Unterseite der Flüssigkeitsfilterkammer (24) und/oder die Ober- und/oder Unterseite der Belüftungsfilterkammer (11) jeweils Stützrippen (18, 26, 27, 36) aufweisen, welche vorzugsweise so angeordnet sind, dass ggf. jeweils gegenüber liegende Stützrippen (18, 26, 27, 36) der Flüssigkeitsfilterkammer (24) oder der Belüftungsfilterkammer (11) schräg und/oder versetzt zueinander verlaufen.

6. Vorrichtung nach einem der vorhergehenden Ansprüche, **dadurch gekennzeichnet, dass** die Flüssigkeitsfilterkammer (24) und/oder die Belüftungsfilterkammer (11) jeweils an ihrer Ober- und/oder Unterseite strahlenförmig verlaufende Vertiefungen (19, 29, 37) aufweisen.

7. Vorrichtung nach einem der vorhergehenden Ansprüche, **dadurch gekennzeichnet, dass** das Gehäuse (10, 20, 30) aus mindestens zwei vorzugsweise übereinander angeordneten Teilen zusammengesetzt ist, wobei besonders bevorzugt ein Gehäuseunterteil (10), ein Gehäusemittelteil (20) und ein Gehäuseoberteil (30) vorgesehen sind.

8. Vorrichtung nach Anspruch 7, **dadurch gekennzeichnet, dass** der Flüssigkeitskanal (6) und/oder der Belüftungskanal (7) in einem vorspringenden zylindrischen Abschnitt (16) eines ersten Teils des Gehäuses, vorzugsweise im Gehäuseunterteil (10), verläuft, wobei der vorspringende zylindrische Abschnitt durch die Belüftungsfilterkammer (11) hindurch geführt ist und in einer entsprechenden Öffnung (23) eines zweiten Teils des Gehäuses, vorzugsweise des Gehäusemittelteils (20), abgedichtet angeordnet ist.

9. Vorrichtung nach Anspruch 7 oder 8, **dadurch gekennzeichnet, dass** das erste Teil, vorzugsweise das Gehäuseunterteil (10), mit dem Einstechdorn (1) verbunden ist, und ein weiteres Teil, vorzugsweise das Gehäuseoberteil (30), mit einem Anschlussgehäuse (40) verbunden ist.

10. Vorrichtung nach Anspruch 9, **dadurch gekennzeichnet, dass** das Anschlussgehäuse ein Ventil, vorzugsweise mit einer verschließbaren Ventilplatte (41) und einem Ventilbetätiger (43), aufweist.

11. Vorrichtung nach einem der vorhergehenden Ansprüche, **dadurch gekennzeichnet, dass** das Gehäuse (10, 20, 30) zumindest abschnittsweise radialsymmetrisch ausgebildet ist.

12. Vorrichtung nach einem der vorhergehenden Ansprüche, **dadurch gekennzeichnet, dass** die Vorrichtung zumindest in den die Flüssigkeit führenden Bereichen aus ABS oder MABS besteht.
